# EUROPEAN PATENT SPECIFICATION

(11) **EP 1 345 586 B1**
(45) Date of publication and mention of the grant of the patent: **09.03.2005**
(21) Application number: 01985387.8
(22) Date of filing: 06.12.2001
(51) Int. Cl.: A61K 7/48

(54) **STABLE SKIN CARE PRODUCT CONTAINING A RETINOID AND A RETINOID BOOSTER SYSTEM IN A DUAL COMPARTMENT PACKAGE**
STABILES HAUTPFLEGEMITTEL DAS EIN RETINOID UND EIN RETINOID-VERSTÄRKENDES SYSTEM ENTHÄLT IN EINER ZWEIKAMMERVERPACKUNG
PRODUIT DE SOIN CUTANE STABLE CONTENANT UN RETINOIDE ET UN SYSTEME ACTIVATEUR DE RETINOIDE DANS UN EMBALLAGE A DEUX COMPARTIMENTS

(30) Priority: 28.12.2000 US 258458 P
(43) Date of publication of application: 24.09.2003
(73) Proprietor: UNILEVER PLC, London EC4P 4BQ (GB); UNILEVER N.V., 3013 AL Rotterdam (NL)
(72) Inventor: GRANGER, Stewart Paton, Unilever Research Colworth, Bedford, Bedfordshire MK44 1LQ (GB); CHANDAR, Prem, Unilever Research U.S. Inc., Edgewater, NJ 07020 (US); SCOTT, Ian Richard, Unilever Research U.S. Inc., Edgewater, NJ 07020 (US)
(74) Representative: Acham, Nicholas Clive
(86) International application number: PCT/EP2001/014485
(87) International publication number: WO 2002/053123

(56) References cited:
- WO-A-02/02074
- US-A- 5 800 596
- US-A- 5 914 116
- US-A- 6 080 393

## Description

The invention relates to stable skin care compositions containing a retinoid and a retinoid booster system in a dual compartment package.

Retinoids (e.g. retinol and retinyl esters) are common ingredients used in cosmetic products. Retinol (vitamin A) is an endogenous compound which occurs naturally in the human body and is essential for normal epithelial cell differentiation. Natural and synthetic vitamin A derivatives have been used extensively in the treatment of a variety of skin disorders and have been used as skin repair or renewal agents. Retinoic acid has been employed to treat a variety of skin conditions, e.g., acne, wrinkles, psoriasis, age spots and discoloration. See e.g. Vahlquist, A. et al., J. Invest. Dermatol., Vol. 94, Holland D.B. and Cunliffe, W.J. (1990), pp. 496-498; Ellis, C.N. et. Al., "Pharmacology of Retinols in Skin," Basel, Karger, Vol. 3, (1989), pp. 249-252; and PCT Patent Application No. WO 93/19743.

Retinol, however, is particularly unstable in cosmetic formulations because retinol can undergo chemical degradation as a consequence of many factors which include oxidation, thermal instability and UV induced degradation. Retinyl esters are also subject to these instabilities although to a lesser extent than retinol.

Retinoid benefits on skin can be enhanced by the coapplication of retinoid booster molecules. However, many if not all of the retinoid booster molecules also increase the instability of the retinol. Therefore, in order to have an effective skin care composition containing both retinoids and retinoid boosting molecules, it is necessary to protect retinoid formulations containing boosters to a higher degree than is necessary for formulations containing retinoids alone.

Therefore, there are problems not only with retinoid stability alone, but also stability of retinoids in the presence of actives which boost the retinoid benefits. Several approaches exist in the prior art which seek to resolve the problem of retinoid stability in cosmetic compositions. For example, multi-compartment systems for delivering compositions have been described in U.S. Patent No. 5,914,216 issued to the assignee of the present invention. In particular, the patent describes two separate containers for separating two different skin actives to provide dual skin benefits with one compartment containing retinoids and the second compartment containing a second active providing a second benefit.

U.S. Patent No. 5,976,555 assigned to Johnson & Johnson discloses skin care compositions comprising oil in water emulsions containing retinoids, an emulsifier system, and a co-emulsifier. The patent describes the use of a container for storing the composition so that the composition is out of contact with oxygen. The container is described for use for the retinoid composition with an emulsifier system and a co-emulsifier alone and does not protect the retinoid from degradation due to contact with retinoid boosters.

U.S. Patent No. 5,800,596 assigned to L'Oreal discloses a water in oil emulsion containing retinol in a dispensing device that has walls impermeable to oxygen or UV light and an oxygen trapping device. The patent does not teach or suggest the use of boosters and the problems associated with retinoid stability in the presence of boosters.

None of the references cited above teach or suggest the need for stabilizing retinoid compositions in the presence of retinoid enhancing actives. Therefore, although dual purpose single formulation cosmetic products have been developed in the prior art, there still remains a need for stable cosmetic compositions that attenuate the existing problems of retinoid stability alone as well as in the presence of retinoid boosters.

The present invention provides a stable skin care product comprising:
a first composition comprising about 0.001% to about 10% of a retinoid selected from the group consisting of retinyl esters, retinol, retinal, and mixtures thereof;
a second composition comprising about 0.0001% to about 50% of at least one retinoid booster selected from the group consisting of citral, citronellol, cocamide DEA, damascenone, 1,3-dimethyl 2 imidazolidinone, geraniol, 18b glycerhetinic acid, 8 OH quinoline, N lauroyl sarcosine, linalool, linoleamide DEA, alpha ionone and linseed oil;
a first compartment for storing the first composition, wherein the first compartment keeps the first composition out of contact with oxygen; and
a second compartment for storing the second composition, the first and second compartments being joined together; thereby avoiding chemical degradation of the first composition that would be caused by contact with the second composition.

The inventive compositions contain a retinoid, which is selected from retinyl esters, retinol, retinal and retinoic acid, preferably retinol or retinyl ester. The term "retinol" includes the following isomers of retinol: all-trans-retinol, 13-cis-retinol, 11-cis-retinol, 9-cis-retinol, 3,4-didehydro-retinol, 3,4-didehydro-13-cis-retinol; 3,4-didehydro-11-cis-retinol; 3,4-didehydro-9-cis-retinol. Preferred isomers are all-trans-retinol, 13-cis-retinol, 3,4-didehydro-retinol, 9-cis-retinol. Most preferred is all-trans-retinol; due to its wide commercial availability.

Retinyl ester is an ester of retinol. The term "retinol" has been defined above. Retinyl esters suitable for use in the present invention are C₁-C₃₀ esters of retinol, preferably C₂-C₂₀ esters, and most preferably C₂, C₃, and C₁₆ esters because they are more commonly available. Examples of retinyl esters include but are not limited to: retinyl palmitate, retinyl formate, retinyl acetate, retinyl propionate, retinyl butyrate, retinyl valerate, retinyl isovalerate, retinyl hexanoate, retinyl heptanoate, retinyl octanoate, retinyl nonanoate, retinyl decanoate, retinyl undecanoate, retinyl laurate, retinyl tridecanoate, retinyl myristate, retinyl pentadecanoate, retinyl heptadeconoate, retinyl stearate, retinyl isostearate, retinyl nonadecanoate, retinyl arachidonate, retinyl behenate, retinyl linoleate, retinyl oleate.

The preferred ester for use in the present invention is selected from retinyl palmitate, retinyl acetate and retinyl propionate, because these are the most commercially available and therefore the cheapest. Retinyl linoleate and retinyl oleate are also preferred due to their efficacy.

Retinol or retinyl ester is employed in the inventive composition in a first composition in an amount of 0.001% to 10%, preferably in an amount of from 0.01% to 1%, most preferably in an amount of 0.01% to 0.5%.

It is believed that retinoids are enzymatically converted in the skin into retinoic acid according to the mechanism described in Chart 1 below.

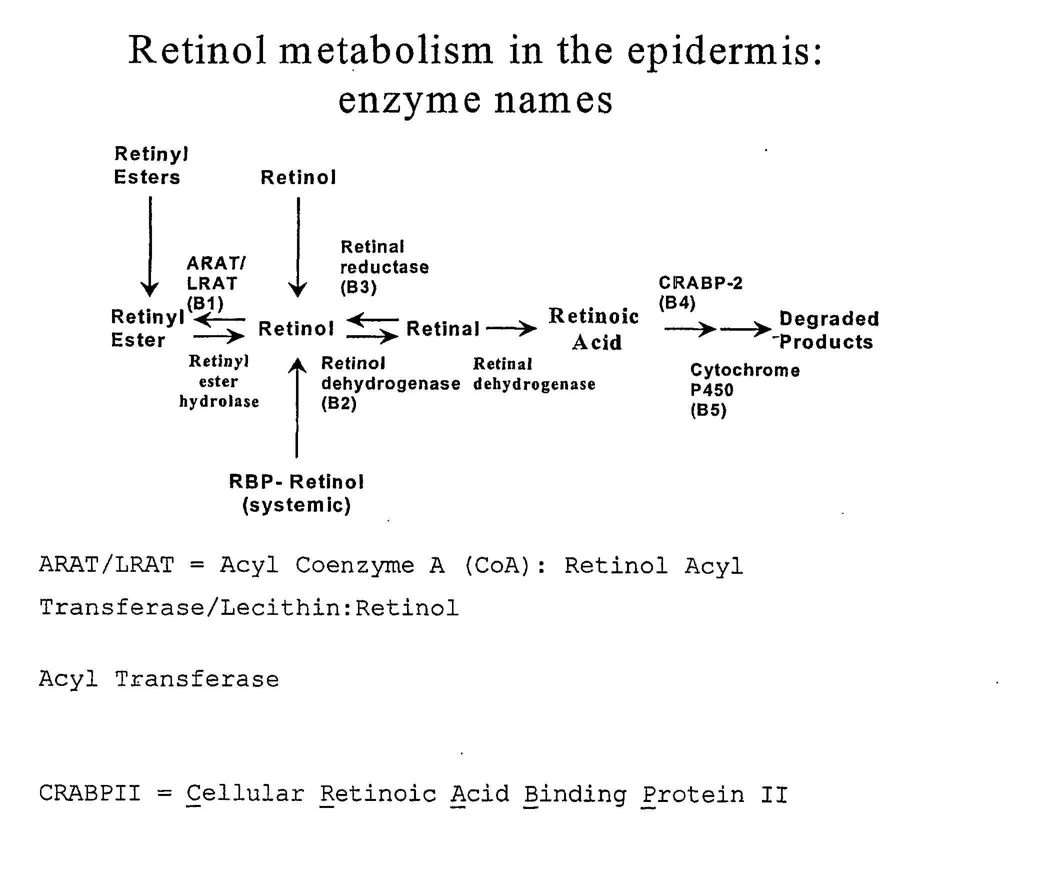

It has been discovered, surprisingly, that certain compounds inhibit ARAT/LRAT, retinal reductase, CRABP11 and retinoic acid oxidation (the latter catalyzed by cytochrome P450 systems), whereas certain other compounds enhance retinol dehydrogenase. The compounds are collectively termed herein as "boosters" and are coded as groups B1 through B5, as can be seen in Chart 1 hereinabove. The boosters, alone or in combination with each other, potentiate the action of a retinoid by increasing the amount of retinol available for conversion to retinoic acid and inhibiting the degradation of retinoic acid. The boosters act in conjunction with a retinoid (e.g. retinol, retinyl ester, retinal, retinoic acid), the latter being present endogenously in the skin. The preferred compositions, however, include a retinoid in the composition, co-present with a booster, to optimize performance.

The present invention includes, in part, a second composition containing 0.0001% to 50%, preferably 0.001% to 10%, most preferably 0.001% to 5% by weight of the composition of at least one booster compound, wherein the compound, either alone or at a combined concentration of 10mM inhibit transglutaminase in an in vivo transglutaminase assay to more than 50%, and a cosmetically acceptable vehicle.

The boosters included in the inventive compositions are selected from the group consisting of:
(a) Two boosters, wherein both are selected from the same group consisting of B2; B3; B4;
(b) binary combinations of boosters selected from the group consisting of
   B1/B2; B1/B3; B1/B4; B1/B5; B2/B3, B2/B4; B2/B5, B3/B4; B3/B5; B4/B5
(c) ternary combinations of boosters selected from the group consisting of
   B1/B2/B3; B1/B2/B4; B1/B2/B5; B1/B3/B4; B1/B3/B5; B1/B4/B5;B2/B3/B4; B2/B3/B5; B2/B4/B5; B3/B4/B5
(d) quaternary combinations of boosters selected from the group consisting of
   B1/B2/B3/B4; B1/B2/B3/ B1/B2/B4/B5; B1/B3/B4/B5; B2/B3/B4/B5; and
(e) a combination of five groups of boosters: B1/B2/B3/B4/B5.

The preferred compositions include at least one booster from the different groups (i.e., groups (b) through (e) above). However, any combination of boosters chosen from the different groups may also be employed in the inventive compositions for desired boosting effects.

The compounds included in the present invention as boosters are first selected based on the ability of such compounds to pass, at a certain concentration listed in Table A, an in-vitro Microsomal Assay for a specific enzyme as described below under sections 2.1 through 2.7. The compound (alone or in combination with another booster) is then subjected to an in vitro transglutaminase assay described below, at an individual or combined concentration of 10 mM. If such combination inhibits transglutaminase to more than 50%, then it is suitable for use in the present invention. If a booster was tested individually, and passes the transglutaminase assay, then it may be combined with another booster or combination that passes the transglutaminase assay.

Preferred compositions according to the present invention contain combinations of boosters which at an individual concentration of 10 mM, inhibit transglutaminase to more than 50%.

The term "conditioning" as used herein means prevention and treatment of dry skin, acne, photodamaged skin, appearance of wrinkles, age spots, aged skin, increasing stratum corneum flexibility, lightening skin color, controlling sebum excretion and generally increasing the quality of skin. The composition may be used to improve skin desquamation and epidermal differentiation.

A booster is a compound which passes an in vitro Microsomal Assay described below in sections 2.1 through 2.7. A compound of the present invention inhibits or enhances at a concentration listed in Table A. An enzyme, to at least a broad % listed in Table A.

**TABLE A**

| Booster Test Concentrations and % Inhibition/Increase | | |
|---|---|---|
| T / LRAT Assay **(To identify B1 boosters)** | | |

| **Invention** | **Compound Concentration** | **% Inhibition** |
|---|---|---|
| | | |
| Broad | 100 µM | > 10% |
| Preferred | 100 µM | > 25% |
| Most Preferred | 100 µM | > 40% |
| Optimum | 100 µM | > 50% |
| Retinol Dehydrogenase Assay **(To identify B2 boosters)** | | |

| **Invention** | **Compound Concentration** | **% Increase** |
|---|---|---|
| | | |
| Broad | 100 µM | > 10% |
| Preferred | 100 µM | > 15% |
| Most Preferred | 100 µM | > 20% |
| Optimum | 100 µM | > 25% |
| Retinal Reductase Assay **(To identify B3 boosters)** | | |

| **Invention** | **Compound Concentration** | **% Inhibition** |
|---|---|---|
| | | |
| Broad | 100 µM | > 5% |
| Preferred | 100 µM | > 10% |
| Most Preferred | 100 µM | > 20% |
| Optimum | 100 µM | > 35% |
| CRABPII Antagonist Assay **(To identify B4 boosters)** | | |

| **Invention** | **Compound : RA Ratio** | **% Inhibition** |
|---|---|---|
| | | |
| Broad | 7000 : 1 | > 25% |
| Preferred | 7000 : 1 | > 50% |
| Most Preferred | 70 : 1 | > 25% |
| Optimum | 70 : 1 | > 50% |
| Retinoic Acid Oxidation Assay **(To identify B5 boosters)** | | |

| **Invention** | **Compound Concentration** | **% Inhibition** |
|---|---|---|
| | | |
| Broad | 100 µM | > 25% |
| Preferred | 100 µM | > 45% |
| Most Preferred | 100 µM | > 70% |
| Optimum | 100 µM | > 80% |

The in vitro Microsomal Assays employed for determining the suitability of the inclusion of the compound in the inventive compositions are as follows:

### 1. Materials

All-trans-retinol, all-trans-retinoic acid, palmitoyl-CoA, dilauroyl phosphatidyl choline, NAD, and NADPH were purchased from Sigma Chemical Company. Stock solutions of retinoids for the microsomal assays were made up in HPLC grade acetonitrile. All retinoid standard stock solutions for HPLC analysis were prepared in ethanol, stored under atmosphere of N2 at -70°C and maintained on ice under amber lighting when out of storage. Other chemicals and the inhibitors were commercially available from cosmetic material suppliers or chemical companies such as Aldrich or International Flavors and Fragrances.

### 2. Methods

### 2.1 Isolation of RPE microsomes (modified from J. C. Saari & D. L. Bredberg, "CoA and Non-CoA Dependent Retinol Esterification in Retinal Pigment Epithelium", J. Bill. Chem. 263, 8084-8090 (1988).

50 frozen hemisected bovine eyecups, with the retina and aqueous humor removed were obtained from W. L. Lawson Co., Lincoln, NE, USA. The eyes were thawed overnight and the colored iridescent membrane was removed by peeling with forceps. Each eyecup was washed with 2x 0.5mL cold buffer (0.1M PO4 / 1mM DTT / 0.25M sucrose, pH 7) by rubbing the darkly pigmented cells with an artist's brush or a rubber policeman. The cell suspension was added to the iridescent membranes and the suspension was stirred for several minutes in a beaker with a Teflon stir bar. The suspension was filtered through a coarse filter (Spectra / Por 925µ pore size polyethylene mesh) to remove large particles, and the resulting darkly colored suspension was homogenized using a Glas-Col with a motor driven Teflon homogenizer. The cell homogenate was centrifuged for 30 min. at 20,000g (Sorvaal model RC-5B centrifuge with an SS34 rotor in 2.5x10cm tubes at 14,000 RPM). The resulting supernatant was subjected to further centrifugation for 60 min. at 150,000g (Beckman model L80 Ultracentrifuge with an SW50.1 rotor in 13x51mm tubes at 40,000 RPM). The resulting pellets were dispersed into -5mL 0.1M PO4 / 5mM DTT, pH 7 buffer using a Heat Systems Ultrasonics, Inc. model W185D Sonifier Cell

Disruptor, and the resulting microsomal dispersion was aliquoted into small tubes and stored at -70°C. The protein concentrations of the microsomes were determined using the BioRad Dye binding assay, using BSA as a standard.

### 2.2 Isolation of rat liver microsomes (R. Martini & M. Murray, "Participation of P450 3A Enzymes in Rat Hepatic Microsomal Retinoic Acid 4-Hydroxylation", Archives Biochem. Biophys. 303, 57-66 (1993).

Approximately 6 grams of frozen rat liver (obtained from Harlan Sprague Dawley rats from Accurate Chemical and Scientific Corp.) were homogenized in 3 volumes of 0.1M tris / 0.1M KCl / 1mM EDTA / 0.25M sucrose, pH 7.4 buffer using a Brinkmann Polytron. The resulting tissue suspension was further homogenized in the motor driven Teflon homogenizer described above. The resulting homogenate was successively centrifuged for 30 min. at 10,000g, 30 min. at 20,000g, and 15 min. at 30,000g, and the resulting supernatant was ultracentrifuged for 80 min. at 105,000g. The pellet was sonicated in -5mL of 0.1M PO4 / 0.1mM EDTA / 5mM MgCl2, pH 7.4 buffer as described above and stored as aliquots at -70°C. Protein concentrations were determined as described above.

### 2.3 Assay for ARAT and LRAT activity (To identify B1)

The procedure below is a modification of a method described in J. C. Saari & D. L. Bredberg, "ARAT & LRAT Activities of Bovine Retinal Pigment Epithelial Microsomes", Methods Enzymol. 190, 156-163 (1990). The following buffer was prepared and stored at 4°C: 0.1M PO4 / 5mM dithiothreitol, pH 7.0 (PO4 / DTT). On the day of the assay, add 2mg BSA per mL of buffer to give a PO4 / DTT / BSA working buffer. 1mM retinol substrate was prepared in acetonitrile and stored in amber bottles under nitrogen gas at -20°C. Solutions of 4mM Palmitoyl-CoA in working buffer (stored in aliquots) and 4mM dilauroyl phosphatidyl choline in ethanol were prepared and stored at -20°C. Inhibitors were prepared as 10mM stock solutions in H2O, ethanol, acetonitrile or DMSO. The quench solution was prepared using pure ethanol containing 50µg/mL butylated hydroxytoluene (BHT), and a hexane solution containing 50µg/mL BHT was used for the extractions.

To a 2 dram glass vial, add the following in order: PO4 / DTT / BSA buffer to give a total volume of 500µL, 5µL acyl donor (4mM palmitoyl-CoA and/or dilauroyl phosphatidyl choline), 5µL inhibitor or solvent blank (10mM stock or further dilutions) followed by approximately 15µg of RPE microsomal protein (approximately 15µL of a ~1mg/mL microsomal protein aliquot). Incubate for 5 min. at 37°C to equilibrate the reaction temperature and then add 5µL 1mM retinol. Cap the vials, vortex for 5 seconds and incubate for 30-90 minutes at 37°C. Quench the reaction by adding 0.5mL ethanol / BHT. Extract the retinoids by adding 3mL hexane / BHT, vortex the tubes for several seconds several times and centrifuge the tubes at low speed for 5 min. to quickly separate the layers. Remove the upper hexane layer into a clean vial, and re-extract the aqueous layer with another 3mL hexane / BHT, as described above. Combine the hexane layers and evaporate the hexane by drying at 37°C under a stream of nitrogen gas on a heated aluminum block. Store the dried residue at -20°C until HPLC analysis. Quantitate the amount of retinyl palmitate and retinyl laurate for ARAT and LRAT activity, respectively, by integration of the HPLC signal as described below.

Note that the incubation solution contains 40µM acyl donor, 100µM or less inhibitor, 10µM retinol, approximately 30µg/mL microsomal protein, and nearly 0.1M PO4, pH 7 / 5mM DTT / 2mg/mL BSA. All steps subsequent to the addition of retinol were done in the dark or under amber lights.

### 2.4 Assay for Retinol Dehydrogenase Activity (To identify B2)

The following stock solutions were prepared:
50mM KH2PO4, pH 7.4 buffer, sterile filtered.
10mM all trans Retinol (Sigma R7632) in DMSO.
200mM Nicotinamide adenine dinucleotide phosphate, sodium salt (NADP) (Sigma N0505) in sterile water.
40mM test compound in appropriate solvent (water, buffer, ethanol, chloroform or DMSO).
1:10 dilution of rat liver Microsomes in 50mM KH2PO4, pH 7.4 buffer (4ug/ul).

In a two-dram glass vial with screw cap, add the following in order:
Buffer to give a final volume of 400µl
25µl diluted Microsomes (final = 100µg) - use boiled Microsomes for controls and regular Microsomes for test samples.
4µl of 200mM NADP (final = 2mM)
1µl of 40mM test compound (final = 100µM)
8µl of 10mM retinol (final = 200µM)

Incubate vials in a 37°C shaking water bath for 45 minutes. Add 500µl ice-cold ethanol to each vial to quench the reaction. Extract the retinoids twice with ice cold hexane (2.7ml per extraction). Retinyl acetate (5µl of a 900µM stock) is added to each vial during the first extraction as a means of monitoring the extraction efficiency in each sample. Samples were vortexed for ten seconds before gently centrifuging for five minutes at 1000rpm, 5°C in a Beckman GS-6R centrifuge. The top hexane layer containing the retinoids is removed from the aqueous layer after each extraction to a clean two-dram vial. Evaporate off the hexane under a gentle stream of nitrogen gas. Store the dried residue at -20°C until HPLC analysis.

### 2.5 Assay for Retinal Reductase Activity (To identify B3)

All stock solution were prepared as above with the following substitutions:
10mM all trans Retinaldehyde (Sigma R2500) in DMSO - instead of retinol.
200mM, Nicotinamide adenine dinucleotide phosphate, reduced form, tetrasodium salt (NADPH) (Sigma N7505) in sterile water - instead of NADP.

In a two-dram glass vial with screw cap, add the following in order:
Buffer to give a final volume of 400µl
25µl diluted Microsomes (final = 100µg) - use boiled Microsomes for controls and regular Microsomes for test samples.
4µl of 200mM NADPH (final = 2mM)
1µl of 40mM test compound (final = 100µM)
3µl of 10mM retinaldehyde (final = 75µM)
Follow the same incubation and extraction procedure as detailed above.

### 2.6 Assay for CRABPII antagonists (To identify B4)

### 2.6.1. Synthesis of CRABPII

### a. System of expression

The gene CRABPII was cloned in pET 29a-c(+) plasmid (Novagen). The cloned gene was under control of strong bacteriophage T7 transcription and translation signals. The source of T7 polymerase was provided by the host cell E.coli BLR (DE3) pLysS (Novagen). The latter has a chromosomal copy of T7 polymerase under lacUV5 control, induced by the presence of IPTG. The plasmid was transferred into E. coli BLR(DE3)pLysS cells by transformation according to the manufacturer protocol (Novagen).

### b. Induction

An overnight culture of the transformed cells was diluted 1:100 into 2xYT containing 50 µg/mL kanamycin and 25µg/mL chloramphenicol. The cells grew while shaking at 37°C until the OD at 600 nm reached 0.6-0.8. Then IPTG was added to a final concentration of 1mM and the culture was incubated for an additional two hours. The cells were harvested by centrifugation at 5000g for 10 minutes at room temperature. The pellet was stored at -20°C.

### 2.6.2. Purification

Purification was performed according to the method described in Norris and Li, 1997.

### a. Lysis

The frozen pellet was thawed at RT and resuspended in 1-2 pellet volumes of freshly prepared lysis buffer (50 mM Tris-Hcl, pH 8, 10%(w/v) sucrose, 1 mM EDTA, 0.05%(w/v) sodium azide, 0.5 mM DTT, 10 mM MnCl2, 2.5 mM phenylmethylsulfonyl fluoride, 2.5 mM benzamidine, 6µg/mL DNase). The lysate was incubated for 30 min at room temperature. Further lysis was accomplished by sonication (six 30-sec bursts at 10,000 psi alternated with five 30-sec delay on ice). The insoluble fraction of the lysate was removed by centrifugation at 15000 rpm 1 hour at 4°C and the supernatant is stored at -20°C.

### b. Gel filtration on Sephacryl S300

The supernatant from step a. was loaded onto a 2.5x100 cm column of sephacryl S-300 (Pharmacia) at room temperature. The elution buffer was 20 mM Tris-HCl, pH 8, 0.5mM DTT, 0.05% sodium azide (buffer A). The flow rate was 2mL/min. Collected 2-mL fractions were checked for ultraviolet absorbance at 280 nm. The fractions representing the peaks were examined by SDS-page for the presence of CRABPII .

### c. Anion-exchange chromatography

2 mL of gel filtration fractions containing CRABPII were loaded onto a quaternary amine anion-exchange column FPLC (Fast Protein Liquid Chromatography) type monoQ (Pharmacia). CRABPII was eluted using a gradient buffer from 100% buffer A to 30% buffer B (100 % buffer B = buffer A + 250 mM NaCl) over a 20-min period at room temperature.1 mL-fractions were collected every minute. Once more, the presence of CRABPII was checked by SDS page. CRABPII was stored at 4°C before freeze-drying using a Micromodulyo 1.5K with vial platform attachment (Edwards High Vacuum International). The desiccated samples were stored at room temperature until their use in the binding assay.

### d. Detection of the presence of CRABPII

The expression and purification of CRABPII was validated using denaturing SDS-polyacrylamide gel electrophoresis (SDS-PAGE) analysis on a 7-15% polyacrylamide gel (Biorad). 10 µL samples were mixed with 10 µL of 2X loading buffer (100 mM Tris-HCl pH6.8, 4% SDS, 0.2% BPB, 20% glycerol, 1mM DTT) and denatured by heating (2 min at 80°C). The samples were loaded onto the gel that was immersed in a 1X Tris-glycine buffer (Biorad) and a constant current (25 mA) was applied for 1 hour at room temperature. After Coomassie blue staining, the protein was identified according to its molecular weight as determinated with the Benchmark prestained protein ladder (Gibco BRL) .

A western blot was used to confirm the presence of CRABPII. The proteins separated on the SDS-PAGE were transferred on an Immobilon-P transfer membrane (Millipore) using a Biorad cassette. The transfer occurred in 1X Tris-glycine buffer (Biorad) + 10% methanol. An electrical current (60 mA) was applied for 3 hours to allow the protein to migrate through the membrane. Afterwards, the membrane was blocked with 5% dry milk in 1X TBS for one hour at room temperature and probed with primary antibodies to CRABPII (1/1000 dilution of mouse anticlonal 5-CRA-B3) in the same buffer at 4°C overnight. The following day, the membrane was washed with PBS (3 x 5 minutes) and then incubated with 1:2000 dilution of the secondary antibody, peroxidase conjugated anti-mouse antibody (ECLTM, Amersham), for 1 hour at room temperature. The membrane was washed with 1xPBS (3x 5 minutes) and the protein was detected using ECL detection kit according to the manufacturer instruction (Amersham).

The concentration of purified CRABPII was determined using BSA kit (Pierce).

### 2.6.3. Radioactive Binding assay

220 pmol of CRABPII was incubated in 20 mM Tris-HCl buffer pH 7.4 with 15 pmol of radioactive all trans retinoic acid (NEN) in a total volume of 70µL. For the competitive assay, another ligand in excess (6670:1, 670:1 or 70:1) was added to the mix. The reaction occurred for one hour at room temperature in the dark. In order to separate the unbound all-trans retinoic acid from the bound all-trans retinoic acid, a 6kD cut-off minichromatography column (Biorad) was used. The storage buffer was discarded using a Microplex manifold for according to the manufacturer instruction (Pharmacia). The samples were loaded onto the column and the separation occurred by gravity over a 30-min period. Retinoic acid ('"RA") bound to CRABPII appeared in the filtrate while free RA remained in the column. The radioactivity of the filtrate was measured by scintillation counter.

### 2.7 Assay for NADPH Dependent Retinoic Acid Oxidation (To identify B5)

The procedure below is a modification of a method described in R. Martini & M. Murray, "Participation of P450 3A Enzymes in Rat Hepatic Microsomal Retinoic Acid 4-Hydroxylation", Archives Biochem. Biophys. 303, 57-66 (1993).

Prepare the following assay buffer and store at 4°C: 0.1M PO4 / 0.1mM EDTA / 5mM MgCl2, pH 7.4. On the day of the assay, prepare a 60mM NADPH solution in buffer. Prepare inhibitor stocks, acidified ethanol / BHT quench solution, and hexane / BHT as described above. A working 1mM retinoic acid solution was prepared by dilution of a 15mM stock (in DMSO) with ethanol.

To a 2 dram vial, add the following in order: assay buffer to give a final volume of 500µL, 20µL 60mM NADPH, 5µL inhibitor or solvent blank, followed by approximately 2mg of rat liver microsomal protein.

Incubate for 5 min. at 37°C, then add 5µL working 1mM retinoic acid solution. Continue incubation for 60min. at 37°C - do not cap the vials, since the oxidation process requires molecular O2 in addition to NADPH. Quench with acidified ethanol / BHT and extract with hexane / BHT as described above. Quantitate the quickly eluting polar retinoic acid metabolites (presumed to be 4-oxo retinoic acid) by integration of the HPLC signal, as described below.

Note that all steps subsequent to the addition of retinoic acid were done in the dark or under amber lights. The final incubation solution contains 2.4mM NADPH, 100µM or less inhibitor, 10µM retinoic acid, approximately 4mg/mL rat liver microsomal protein and nearly 0.1M PO4 / 0.1mM EDTA / 5mM MgCl2.

### HPLC analysis of individual retinoids

Samples for retinoid quantitation by HPLC were prepared by dissolving the residue in each vial with 100µL of methanol. The solution was transferred to a 150µL glass conical tube within a 1mL shell vial, capped tightly, and placed inside a Waters 715 Autosampler. Aliquots of 60µL were injected immediately and analyzed for retinoid content.

The chromatography instrumentation consisted of a Waters 600 gradient controller / pump, a Waters 996 Photodiode Array detector and a Waters 474 Scanning Fluorescence detector. Two HPLC protocols were used for retinoid analysis. For the ARAT and LRAT assay, the separation of retinol and retinol esters was performed with a Waters 3.9x300mm C18 Novapak reverse-phase analytical column and Waters Sentry NovaPak C18 guard column with an 80:20(v/v) methanol / THF isocratic mobile phase adjusted to a flow rate of 1mL/min. for 10 min. The eluate was monitored for absorbance at 325nm and fluorescence at 325ex/480em. A shorter Waters 3.9x150mm C18 Novapak reverse-phase analytical column and Waters Sentry NovaPak C18 guard column were used to separate retinoid acids and alcohols for the retinol and retinoic acid oxidation assays utilizing a modification of a gradient system described by A. B. Barua, "Analysis of Water-Soluble Compounds: Glucuronides", Methods Enzymol. 189, 136-145 (1990). This system consisted of a 20 min. linear gradient from 68:32(v/v) methanol/ water containing 10mM ammonium acetate to 4:1(v/v) methanol:dichloromethane followed by a 5 min. hold at a flow rate of 1mL/min.. The column eluate was monitored from 300nm to 400nm.

These protocols were selected based on their ability to clearly resolve pertinent retinoid acids, alcohols, aldehydes, and/or esters for each assay and relative quickness of separation. Identification of individual retinoids by HPLC was based on an exact match of the retention time of unknown peaks with that of available authentic retinoid standards and UV spectra analysis (300-400nm) of unknown peaks against available authentic retinoids.

The boosters suitable for further testing in the transglutaminase assay include but are not limited to the boosters listed in Tables B1 through B5 below.

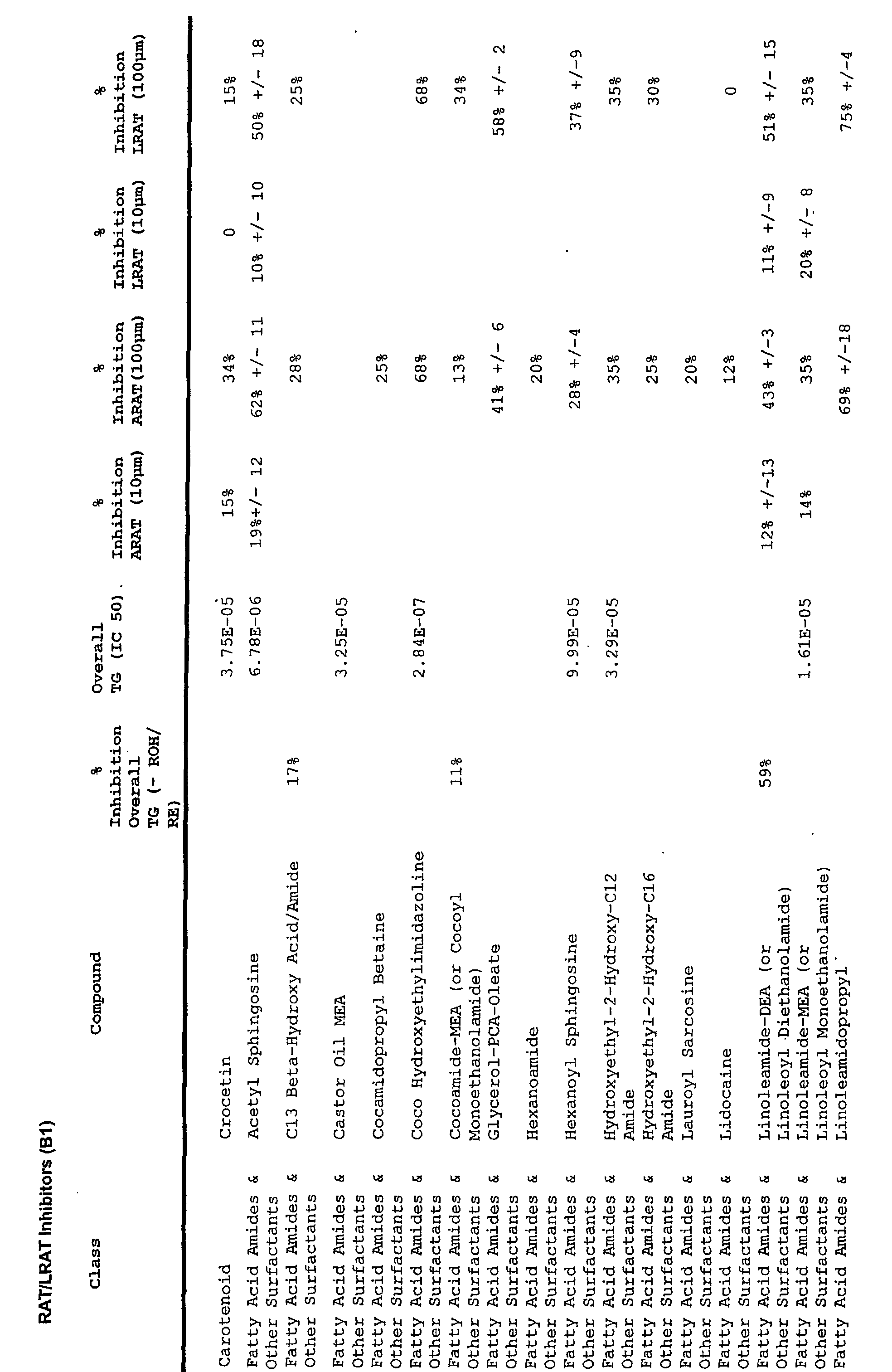

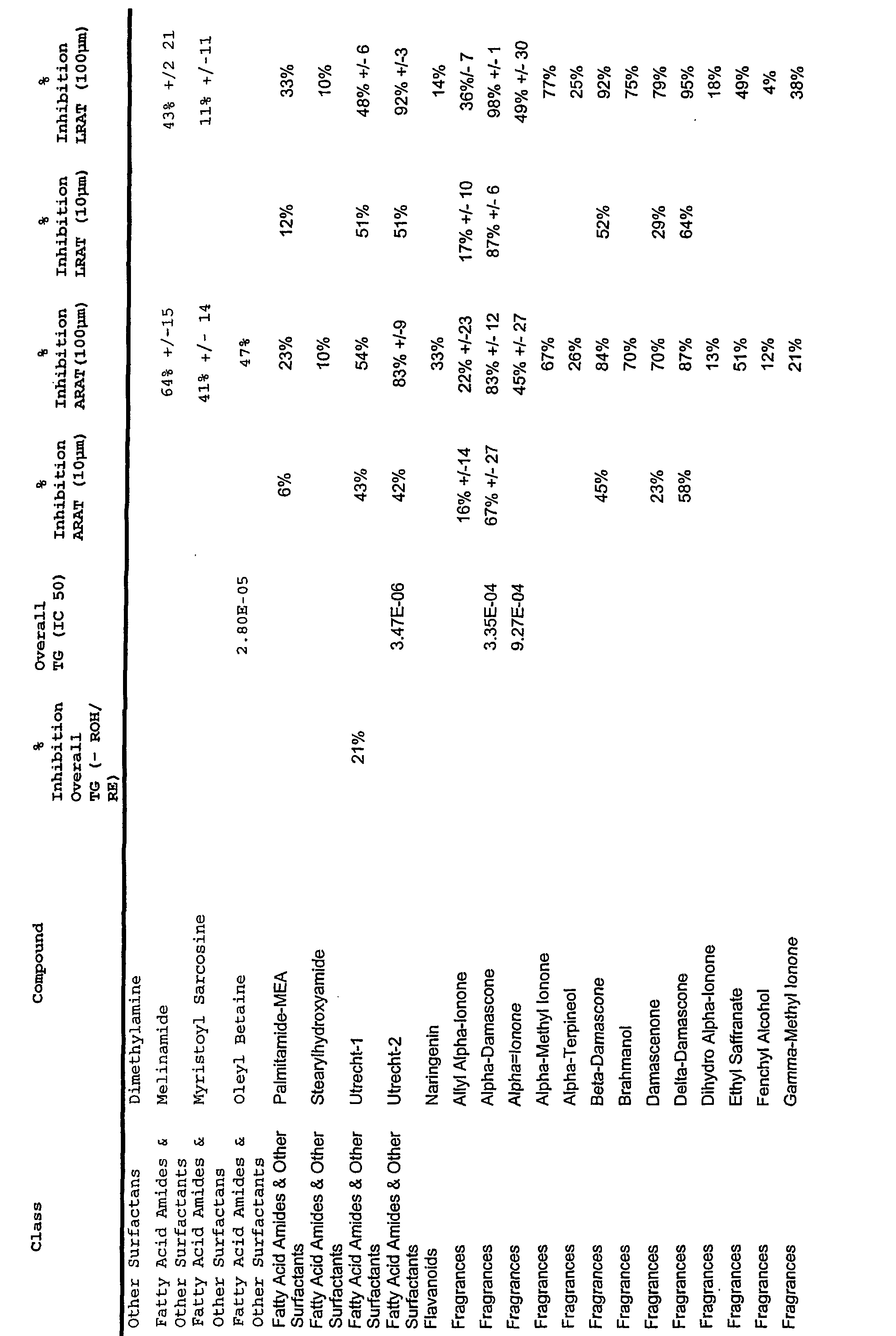

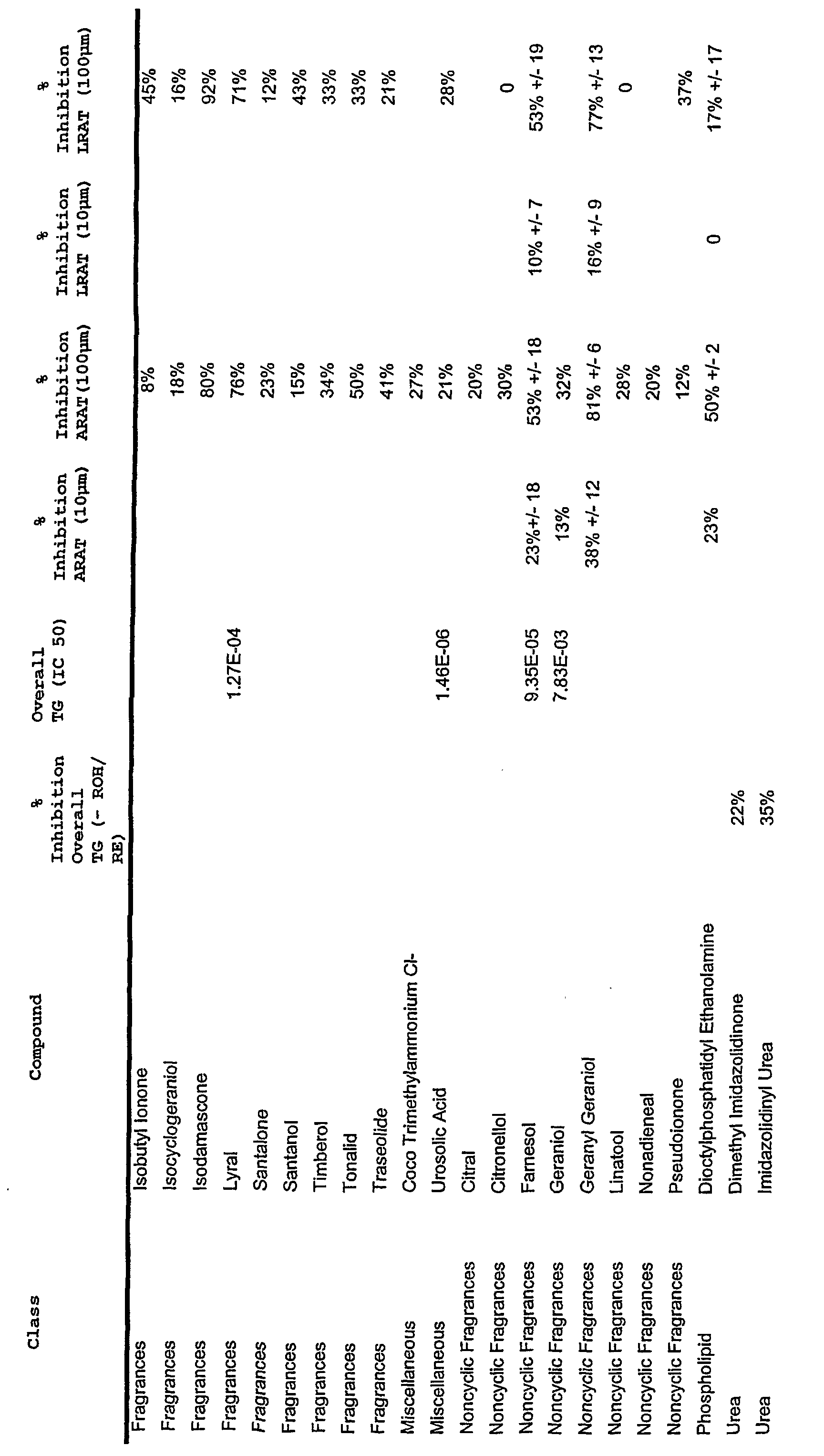

| **Retinol Dehydrogenase Activators (B2)** | | | |
|---|---|---|---|
| **Class** | **Compound** | **% Increase Retinol Dehydrogenase** | |
| Phospholipid | Phosphatidyl Choline | 21% increase | |
| Phospholipid | Sphingomyelin | 26% increase | |
| **Retinaldehyde Reductase Inhibitors (B3)** | | | |

| Class | Compound | Overall TG (IC 50) | % Inhibition Retinal Reductase |
|---|---|---|---|
| Aldehyde | Vanillin | 9.70E-03 | 6% |
| Fatty Acid | Arachidic Acid | | 20% |
| Fatty Acid | Arachidic Acid | | 49% |
| Fatty Acid | Linoleic Acid | 1.63E-04 | 62% +/-2 |
| Fatty Acid | Linolenic Acid | 1.34E-04 | 54% +/-16 |
| Fatty Acid | Myristic Acid | 1.72E-05 | 26% |
| Miscellaneous | Amsacrine | 6.26E-06 | 22% + /-8 |
| Miscellaneous | Carbenoxolone | 3.61E-07 | 26% +/-2 |
| Miscellenous | Glycyrretinic Acid | 8.64E-06 | 38% =/- 1 |
| Phospholipid | Phosphatidyl ethanolamine | | 37% |
| **CRABPII Antagonists (B4)** | | | |

| **Class** | **Compound** | **Overall TG (IC 50)** | **% Inhibition CRABPII** |
|---|---|---|---|
| Fatty Acid | Elaidic Acid | 6.50E-05 | >50% |
| Fatty Acid | Hexadecanedioic Acid | 1.30E-04 | >50% |
| Fatty Acid | 12-Hydroxystearic Acid | 2.91E-05 | >50% |
| Fatty Acid | Isostearic Acid | 6.88E-05 | >50% |
| Fatty Acids | Linseed Oil | | >50% |

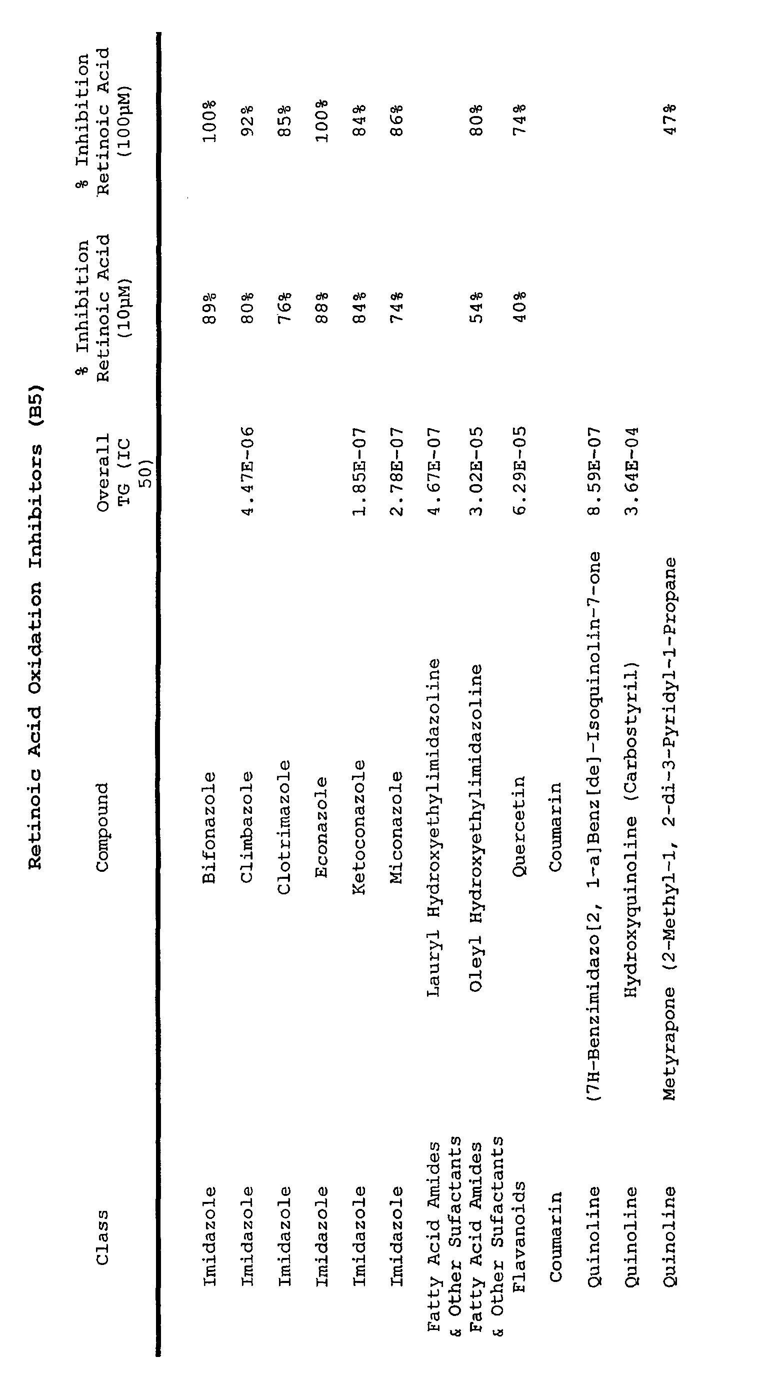

The boosters or combinations thereof inhibit transglutaminase (hereinafter "Tgase") in a transglutaminase assay described below to at least 50% at a concentration of 10mM.

| TGase Assay | | |
|---|---|---|
| **Invention** | **Compound Concentration** | **% Inhibition** |
| | | |
| Broad | 10 mM | > 50% |
| Preferred | 1 mM | > 50% |
| Most Preferred | 100 M | > 50% |
| Optimum | 10 M | > 50% |

### Transglutaminase Assay and Keratinocyte Differentiation

During the process of terminal differentiation in the epidermis, a 15nm thick layer of protein, known as the cornified envelope (CE) is formed on the inner surface of the cell periphery. The CE is composed of numerous distinct proteins which have been cross-linked together by the formation of N^{ε}*-*(Y-glutamyl) lysine isodipeptide bonds catalyzed by the action of at least two different transglutaminases (TGases) expressed in the epidermis. TGase I is expressed in abundance in the differentiated layers of the epidermis, especially the granular layer, but is absent in the undifferentiated basal epidermis. Thus TGase I is a useful marker of epidermal keratinocyte differentiation with high TGase I levels indicating a more differentiated state. An ELISA based TGase I assay, using a TGase I antibody, was used to assess the state of differentiation of the cultured keratinocytes in the examples that follow.

Keratinocytes (cultured as described above) were plated in 96 well plates at a density of 4,000-5,000 cells per well in 200µl media. After incubation for two to three days, or until cells are ~50% confluent, the media was changed to media containing test compounds (five replicates per test). The cells were cultured for a further 96 hours after which time the media was aspirated and the plates stored at -70°C. Plates were removed from the freezer, and the cells were washed twice with 200µl of 1x PBS. The cells were incubated for one hour at room temperature (R/T) with TBS/5% BSA (wash buffer, bovine serum albumin). Next the TGase primary antibody was added: 50µl of monoclonal anti-Tgase I Ab B.C. diluted 1:2000 in wash buffer. The primary antibody was incubated for 2 hours at 37°C and then rinsed 6x with wash buffer. Cells were then incubated with 50µl of secondary antibody (Fab fragment, peroxidase conjugated anti-mouse IgG obtaining from Amersham) diluted 1:4,000 in wash buffer for two hours at 37°C, then rinsed three times with wash buffer. Following the rinse with washing buffer, the cells were rinsed 3x with PBS. For colourimetric development, the cells were incubated with 100µl substrate solution (4 mg o-phenylenediamine and 3.3 µl 30% H₂O₂ in 10ml 0.1M citrate buffer pH 5.0) for exactly five minutes, R/T, in darkness (under aluminum foil). The reaction was stopped by the addition of 50µl 4N H₂SO₄. The absorbance of samples was read at 492nm in a 96 well plate UV spectrophotometer. Out of the five replicates, four were treated with both antibodies, the fifth one was use as a Tgase background control. TGase levels were determined and expressed as percentage control.

Transglutaminase levels were determined and expressed in the Tables B1 through B5 above either as:
(i) % (booster + retinol inhibition / control inhibition) - % (ROH inhibition / control inhibition), which measures the added effect of booster + retinol induced TGase inhibition over retinol alone, or
or (ii) as an IC50 value when the inhibitory effect of multiple booster concentrations was examined - this provides the concentration of booster which, in combination with a constant retinol concentration of 10⁻⁷M, inhibits TGase by 50%.

It is the IC50 value that is used as a benchmark in the present invention.

### Best Groups of Boosters for testing in Transglutaminase assay

### B1 Compounds

| | |
|---|---|
| 1. Fatty Acid Amides | These are readily commercially available and have the added advantage of being surfactants and thus help generate emulsions suitable for cosmetic preparations. |
| 2. Ceramides | These can additionally act as precursors of stratum corneum barrier ceramides. |
| 3. Carotenoids | These can offer some UV protection and act as natural colorants. |
| 4. Flavanoids | Natural antioxidants. |
| 5. Cyclic fragrances | These are readily commercially available and additionally can be used to fragrance the product. |
| 6. Non-cyclic fragrances | These can be used to fragrance the product. |
| 7. Phospholipid analogues | These can be utilised by skin cells to nourish the generation of barrier components. |
| 8. Ureas | These are readily commercially available and can also act as preservatives for the product. |

### B2 Compounds

| | |
|---|---|
| 1. Phosphatidyl choline | Most preferred as most active activator of Retinol Dehydrogenase |
| 2. Sphingomyelin | |

### B3 Compounds

| | |
|---|---|
| Arachidonic Acid Linoleic Acid Linolenic Acid Myristic Acid | Fatty Acids which can be useful in maintaining stratum corneum barrier |
| Linoleic Acid Linolenic Acid | Essential Fatty Acids |
| Arachidonic Acid Myristic Acid | Non-essential fatty acids |
| Glycyrrhetinic Acid | Polycyclic triterpene carboxylic acid which is readily obtained from plant sources. |
| Phosphatidyl ethanolamine | Can be incorporated into cellular membranes. |

### B4 Compounds

| | |
|---|---|
| Hexadecanedioic acid 12-hydroxystearic acid Isostearic acid | Saturated fatty acids. |
| Linseed oil Elaidic acid | Unsaturated fatty acids |
| Elaidic acid Isostearic acid Hexadecanedioic acid | Solid at room temperature |
| Linseed oil 12-hydroxystearic acid | Liquid at room temperature |

### B5 Compounds

| | |
|---|---|
| Bifonazole Climbazole Clotrimazole Econazole Ketoconazole Miconazole | Antimicotics |
| Climbazole | Readily commercially available |
| Lauryl hydroxyethylimidazoline | Compounds which are readily commercially available and have the added advantage of being surfactants and thus help generate emulsions suitable for cosmetic preparations. |
| Quercetin | Naturally occuring flavanoid which has antioxidant properties. |
| Coumarin | Natural colorant |
| Quinolines Isoquinolines | |
| Metyrapone | |

### DUAL COMPARTMENT PACKAGE

As discussed hereinabove, compositions which include retinoids are generally unstable and may undergo chemical degradation. Moreover, it has been surprisingly found that boosters, although beneficial for enhancing the retinoid benefits, also contribute to the chemical instability of retinoids. The booster induced retinol destabilization dramatically reduces the overall efficacy of the boosted retinoid composition when both ingredients are contained in a single formula.
Therefore, in order to protect against retinoid breakdown while still providing the beneficial effects of retinoid boosters, the present invention provides a dual compartment package that contains a first composition containing retinoids in a first compartment and a second composition containing at least one retinoid booster in a second compartment. The first composition provides a first benefit to the skin while the second composition works to boost or enhance the effect of the first benefit.

The dual compartment package may be designed in various ways known to persons of ordinary skill in the art as long as the purpose of providing the first and second compositions in two separate containers is achieved. In one embodiment, the dual compartment package is in the form of two jars or bottles adjoiningly attached. In a second embodiment, the dual compartment package is in the form of a single bottle/jar with a division separating an interior of the bottle/jar into a first and second compartment. Other embodiments are contemplated as being within the scope of the present invention as long as the compositions are retained separately.

### MINIMAL OXYGEN PERMEABLE FIRST COMPARTMENT

As discussed hereinabove, retinoid compositions are prone to degradation in the presence of oxygen. Therefore, the present invention provides a first compartment of the dual compartment package that is minimally permeable to oxygen to aid in maintaining a stabilized first composition. The first composition comprising retinoids is then kept out of contact with oxygen prior to application by a user.

The minimal oxygen permeable compartment can be constructed in various methods known to persons of ordinary skill in the art. Specifically, the inventive compositions should not be in direct contact with oxygen or air, and oxygen should be prevented from seeping through the outer walls of the package. Packages which are opaque to light and impermeable to oxygen may be used. For example, aluminum may be used for the walls of the package, or as lining inside the package.

In an additional embodiment, both the first and second compartments are constructed to be minimally permeable to oxygen to attenuate the degradation of both the first and second compositions.

### Cosmetically Acceptable Vehicle

The product according to the present invention also comprises a cosmetically acceptable vehicle to act as a dilutant, dispersant, or carrier for the active components in the either or both the first and second compositions, so as to facilitate their distribution when the composition is applied to the skin.

Vehicles other than or in addition to water can include liquid or solid emollients, solvents, humectants, thickeners and powders. An especially preferred nonaqueous carrier is a polydimethyl siloxane and/or a polydimethyl phenyl siloxane. Silicones of this invention may be those with viscosities ranging anywhere from about 10 to 10,000,000 centistokes at 25°C. Especially desirable are mixtures of low and high viscosity silicones. These silicones are available from the General Electric Company under trademarks Vicasil, SE and SF and from the Dow Corning Company under the 200 and 550 Series. Amounts of silicone which can be utilized in the compositions of this invention range anywhere from 5 to 95%, preferably from 25 to 90% by weight of the composition.

### Optional Skin Benefit Materials and Cosmetic Adjuncts

In either one or both of the first and second compositions of the present invention, an oil or oily material may be present, together with an emulsifier to provide either a water-in-oil emulsion or an oil-in-water emulsion, depending largely on the average hydrophilic-lipophilic balance (HLB) of the emulsifier employed.

Various types of active ingredients may be present in either one or both of the first and second cosmetic compositions of the present invention and are described below. Actives are defined as skin or hair benefit agents other than emollients and other than ingredients that merely improve the physical characteristics of the composition. Although not limited to this category, general examples include sunscreens, skin lightening agents, tanning agents.

Sunscreens include those materials commonly employed to block ultraviolet light. Illustrative compounds are the derivatives of PABA, cinnamate and salicylate. For example, octyl methoxycinnamate and 2-hydroxy-4-methoxy benzophenone (also known as oxybenzone) can be used. Octyl methoxycinnamate and 2-hydroxy-4-methoxy benzophenone are commercially available under the trademarks, Parsol MCX and Benzophenone-3, respectively.

The exact amount of sunscreen employed in the emulsions can vary depending upon the degree of protection desired from the sun's UV radiation.

Another preferred optional ingredient is selected from essential fatty acids (EFAs), i.e., those fatty acids which are essential for the plasma membrane formation of all cells, in keratinocytes EFA deficiency makes cells hyperproliferative. Supplementation of EFA corrects this. EFAs also enhance lipid biosynthesis of epidermis and provide lipids for the barrier formation of the epidermis. The essential fatty acids are preferably chosen from linoleic acid, Y-linolenic acid, homo- Y-linolenic acid, columbinic acid, eicosa-(n-6,9,13)-trienoic acid, arachidonic acid, Y-linolenic acid, timnodonic acid, hexaenoic acid and mixtures thereof.

Emollients are often incorporated into cosmetic compositions of the present invention. Levels of such emollients may range from about 0.5% to about 50%, preferably about 5% and 30% by weight of the total composition. Emollients may be classified under such general chemical categories as esters, fatty acids and alcohols, polyols and hydrocarbons.

Esters may be mono- or di-esters. Acceptable examples of fatty di-esters include dibutyl adipate, diethyl sebacate, diisopropyl dimerate, and dioctyl succinate. Acceptable branched chain fatty esters include 2-ethyl-hexyl myristate, isopropyl stearate and isostearyl palmitate. Acceptable tribasic acid esters include triisopropyl trilinoleate and trilauryl citrate. Acceptable straight chain fatty esters include lauryl palmitate, myristyl lactate, oleyl eurcate and stearyl oleate. Preferred esters include coco-caprylate/caprate (a blend of coco-caprylate and coco-caprate), propylene glycol myristyl ether acetate, diisopropyl adipate and cetyl octanoate.

Suitable fatty alcohols and acids include those compounds having from 10 to 20 carbon atoms. Especially preferred are such compounds such as cetyl, myristyl, palmitic and stearyl alcohols and acids.

Among the polyols which may serve as emollients are linear and branched chain alkyl polyhydroxyl compounds. For example, propylene glycol, sorbitol and glycerin are preferred. Also useful may be polymeric polyols such as polypropylene glycol and polyethylene glycol. Butylene and propylene glycol are also especially preferred as penetration enhancers.

Exemplary hydrocarbons which may serve as emollients are those having hydrocarbon chains anywhere from 12 to 30 carbon atoms. Specific examples include mineral oil, petroleum jelly, squalene and isoparaffins.

Another category of functional ingredients within the cosmetic compositions of the present invention are thickeners. A thickener will usually be present in amounts anywhere from 0.1 to 20% by weight, preferably from about 0.5% to 10% by weight of the composition. Exemplary thickeners are cross-linked polyacrylate materials available under the trademark Carbopol from the B.F. Goodrich Company. Gums may be employed such as xanthan, carrageenan, gelatin, karaya, pectin and locust beans gum. Under certain circumstances the thickening function may be accomplished by a material also serving as a silicone or emollient. For instance, silicone gums in excess of 10 centistokes and esters such as glycerol stearate have dual functionality.

Powders may be incorporated into one or both of the first and second cosmetic compositions of the cosmetic product of the present invention. These powders include chalk, talc, Fullers earth, kaolin, starch, smectite clays, chemically modified magnesium aluminum silicate, organically modified montmorillonite clay, hydrated aluminum silicate, fumed silica, aluminum starch octenyl succinate and mixtures thereof.

Other adjunct minor components may also be incorporated into one or both of the first and second compositions of the cosmetic product of the present invention. These ingredients may include coloring agents, opacifiers and perfumes. Amounts of these materials may range anywhere from 0.001% up to 20% by weight of the composition.

The first and second compositions of the cosmetic product of the present invention are intended primarily as a product for topical application to human skin, especially as an agent for conditioning and smoothing the skin, and preventing or reducing the appearance of wrinkled or aged skin.

In use, a small quantity of the first composition, for example from 1 to 5ml, is applied to exposed areas of the skin, from a suitable container or applicator and, if necessary, it is then spread over and/or rubbed into the skin using the hand or fingers or a suitable device. Simultaneously, a small quantity of the second composition, for example from 1 to 5 ml, is applied to exposed areas of the skin, from a suitable container or applicator and, if necessary, it is also spread over and/or rubbed into the skin using the hand or fingers or a suitable device. Therefore, depending upon the intensity of treatment benefits desired, the first and second compositions may be used alone, simultaneously, or in consecutive order.

### Product Form and Packaging

The topical skin treatment composition of the invention can be formulated as a lotion, a fluid cream, a cream or a gel.

### Methods

Retinol (50% in tween 80) was dissolved in approximately 50% aqueous ethanol to provide a solution giving an OD at 360nm of approximately 0.6 when measured in a 200 µl volume in a 96 well plate using a standard 96 well spectrophotometer.

Booster molecules were added at approximately 0.1% concentration and the OD 360 measured as above immediately and after 60 hours at room temperature in the dark. A correction was applied to the OD after 60 hours (divide by 0.85) to account for increased concentration of the retinol due to evaporation of solvent from the plate.

### Results

| **BOOSTER** | **FOLD INCREASE IN RATE OF RETINOL LOSS** |
|---|---|
| CITRAL | 3.1 |
| CITRONELLOL | 1.5 |
| COCAMIDE DEA | 1.9 |
| COUMARIN | 1.4 |
| DAMASCONE | 3.7 |
| 1,3 DIMETHYL 2 IMIDAZOLIDINONE | 1.4 |
| GERANIOL | 1.3 |
| 18b GLYCERHETINIC ACID | 1.6 |
| 8 OH QUINOLINE | 1.5 |
| N LAURY SARCOSINE | 2.6 |
| LINALOOL | 2.0 |
| LINOLEAMIDE DEA | 3.0 |
| LINOLEIC ACID | 3.4 |
| ALPHA IONONE | 1.3 |
| LINSEED OIL | 1.5 |

The Boosters tested caused marked increases in the instability of the retinol.

This will make it necessary to use formulation/packaging options providing considerably better stability to the retinol when boosters are used compared to those needed for retinol alone.

### EXAMPLE 1

To establish whether synergistic inhibition of transglutaminase expression occurred by combinations of B1 and B5 active compounds with retinol, it is essential to determine the dose response profiles (including IC50 values) of the active compounds when tested individually in the presence of retinol. This data was used to determine an appropriate sub-maximal inhibitory concentration of each active compound, to make it possible to identify synergistic effects of mixtures of the active compounds in the presence of retinol. In order to demonstrate synergy of two compounds, it is essential to select concentrations to test that are at most IC20, in other words a compound concentration that individually boosts the retinol inhibition of transglutaminase expression by 20%. Two such compounds should have an additive inhibition of 40%. Using this strategy to determine concentration leaves a window of 40-100% for further transglutaminase inhibition for detecting synergy of the two compounds under examination. A more challenging concentration criteria would be selecting concentrations of compounds which alone showed no boosted retinol inhibition of transglutaminase. In this study however we chose an even more challenging criteria. We selected concentrations of compounds that were 10 fold and 100 fold lower than the minimally effective transglutaminase inhibiting concentration. Identification of synergistic combinations using such very low concentrations would mean that the most effective synergistic combinations were identified.

The data in the following table represents the concentrations of compound that are 2 logs lower than the minimally inhibitory compound concentration. These were the concentrations used in the B2/B5 combination studies.

| Compound | Concentration |
|---|---|
| | |

| B1 Compounds | |
|---|---|
| Linoleoyl monoethanolamide | 1.00E-06 |
| Palmitamide monoethanolamide | 1.00E-06 |
| Farnesol | 3.16E-06 |
| Hexyl sphingosine | 1.00E-06 |
| Utrecht-2 | 3.16E-08 |
| Oleoyl betaine | 3.16E-07 |
| Oleoyl hydroxyethylimidazoline | 1.00E-08 |
| Cocoyl hydroxyethylimidazoline | 1.00E-09 |
| Ursolic acid | 1.00E-08 |
| Alpha-ionone | 3.16E-05 |
| | |

| B5 Compounds | |
|---|---|
| Ketoconazole | 1.00E-09 |
| Miconazole | 3.16E-09 |
| Climbazole | 1.00E-08 |
| Amino benzotriazole | 1.00E-06 |
| 3,4-dihydroquinoline | 1.00E-06 |
| 2-hydroxyquinoline | 3.16E-06 |
| | |

To investigate synergistic inhibition of transglutaminase expression by combinations of B1 and B5 active compounds with retinol, selected combinations of compounds were tested at concentrations given in the above table. The following data was obtained:

| Combination | B1 Compound | B2 Compound | Mean % control TGase |
|---|---|---|---|
| | | | |
| **B1/B5** | **Farnesol** | **Ketoconazole** | **84%** |
| **B1/B5** | **Hexanoyl sphingosine** | **Miconazole** | **68%** |
| **B1/B5** | **Hexanoyl sphingosine** | **Ketoconazole** | **64%** |
| **B1/B5** | **Hexanoyl sphingosine** | **3,4-dihydroquinoline** | **89%** |
| **B1/B5** | **Hexanoyl sphingosine** | **Aminobenzotriazole** | **81%** |
| **B1/B5** | **Hexanoyl sphingosine** | **Climbazole** | **63%** |
| **B1/B5** | **Oleoyl betaine** | **Ketoconazole** | **81%** |
| **B1/B5** | **Oleoyl hydroxyethylimidazoline** | **Climbazole** | **52%** |
| **B1/B5** | **Cocoyl hydroxyethylimidazoline** | **Climbazole** | **71%** |
| **B1/B5** | **Ursolic acid** | **2-hydroxyquinoline** | **74%** |
| **B1/B5** | **Alpha-ionone** | **Miconazole** | **84%** |
| **B1/B5** | **Alpha-ionone** | **Ketoconazole** | **82%** |
| **B1/B5** | **Alpha-ionone** | **2-hydroxyquinoline** | **76%** |
| **B1/B5** | **Utrecht-2** | **aminobenzotriazole** | **82%** |
| | | | |
| B1/B5 | Linoleoyl monoethanolamide | Ketoconazole | 93% |
| B1/B5 | Linoleoyl monoethanolamide | Climbazole | 94% |
| B1/B5 | Naringenin | Ketoconazole | 100% |
| B1/B5 | Quercetin | Climbazole | 92% |
| B1/B5 | Castor Oil monoethanolamide | Climbazole | 98% |
| B1/B5 | Castor Oil monoethanolamide | Clotrimazole | 100% |
| | | | |

The efficacy of the B1/B5 combinations splits into two classes - particularly effective combinations (bolded in the above table) and barely effective combinations (not bolded). It was unexpected that certain B1/B5 combinations performed better than other combinations. Those combinations which were barely effective were (i) fatty acid amides + azoles (ii) hydroxy fatty acid amides + azoles and (iii) naringenin/quercetin + azoles. The effective combinations contained B1 boosters combined with B5 boosters from the following classes: fatty hydroxyethyl imidazoline surfactants, cyclic aliphatic unsaturated compounds, polycyclic triterpenes, n-substituted fatty acid amides.

## Claims

1. A stable skin care product comprising:
a first composition comprising 0.001% to 10% of a retinoid selected from the group consisting of retinyl esters, retinol, retinal, and mixtures thereof;
a second composition comprising 0.0001% to 50% of at least one retinoid booster selected from the group consisting of citral, citronellol, cocamide DEA, damascenone, 1,3-dimethyl 2 imidazolidinone, geraniol, 18b glycerhetinic acid, 8 OH quinoline, N lauroyl sarcosine, linalool, linoleamide DEA, alpha ionone and linseed oil;
a first compartment for storing the first composition, wherein the first compartment keeps the first composition out of contact with oxygen; and
a second compartment for storing the second composition, the first and second compartments being joined together; thereby avoiding chemical degradation of the first composition that would be caused by contact with the second composition.

2. The stable skin care product of Claim 1 wherein the second composition has at least two retinoid boosters in an amount of 0.0001% to 50%.

3. The stable skin care product of Claims 1 or Claim 2, wherein the retinoid provides a first benefit and the retinoid booster boosts the first benefit.

4. The stable skin care product of any one of Claims 1 to 3, wherein the first compartment comprises an aluminium layer.

5. The stable skin care product of any one of Claims 1 to 4, wherein the second compartment keeps the second composition out of contact with oxygen.

6. The stable skin care product of Claim 5, wherein the second compartment comprises an aluminium layer.

7. The stable skin care product of any preceding claims wherein the first composition comprises about 0.01% to about 1% of a retinoid selected from the group consisting of retinyl esters, retinol, retinal and mixtures thereof.

8. The stable skin care product of any preceding claim, wherein the retinoid booster is climbazole.

9. A method of conditioning skin, the method comprising applying topically to the skin the product of any one of Claims 1 to 8.

10. A method of mimicking the effect on skin of retinoic acid, the method comprising applying to the skin the product of any one of Claims 1 to 8.

11. Use of the stable skin care product of any one of Claims 1 to 8 in the preparation of a medicament for conditioning skin.

12. The stable skin care product of any one of Claims 1 to 8 for conditioning skin.

## Patentansprüche

1. Stabiles Hautpflegeprodukt, umfassend:
- eine erste Zusammensetzung, die 0,001 % bis 10 % eines Retinoids umfaßt, ausgewählt aus der Gruppe, bestehend aus Retinyiestern, Retinol, Retinall und Gemischen hiervon;
- eine zweite Zusammensetzung, die etwa 0,0001 % bis etwa 50 % von zumindest einem Retinoidverstärker umfaßt, ausgewählt aus der Gruppe, bestehend aus Citral, Citronellol, Cocamid DEA, Damascenon, 1,3-Dimethyl-2-imidazolidinon, Geraniol, 18b-Glycyrrhetinsäure, 80H-Chinolin, N-Lauroylsarkosin, Linalool, Linoleamid DEA, alpha-Ionon und Leinsamenöl;
- eine erste Kammer zur Lagerung der ersten Zusammensetzung, wobei die erste Kammer die erste Zusammensetzung fern von einem Kontakt mit Sauerstoff hält; und
- eine zweite Kammer zur Lagerung der zweiten Zusammensetzung, wobei die erste und die zweite Kammer miteinander verbunden sind, wodurch die chemische Zersetzung der ersten Zusammensetzung vermieden wird, die durch den Kontakt mit der zweiten Zusammensetzung verursacht werden würde.

2. Stabiles Hautpflegeprodukt nach Anspruch 1, wobei die zweite Zusammensetzung zumindest zwei Retinoidverstärker in einer Menge von 0,0001 bis 50 Gew.-% aufweist.

3. Stabiles Hautpflegeprodukt nach Anspruch 1 oder Anspruch 2, wobei das Retinoid einen ersten Nutzen liefert, und der Retinoidverstärker den ersten Nutzen verstärkt.

4. Stabiles Hautpflegeprodukt nach einem der Ansprüche 1 bis 3, wobei die erste Kammer eine Aluminiumschicht umfaßt.

5. Stabiles Hautpflegeprodukt nach einem der Ansprüche 1 bis 4, wobei die zweite Kammer die zweite Zusammensetzung von Sauerstoff fernhält.

6. Stabiles Hautpflegeprodukt nach Anspruch 5, wobei die zweite Kammer eine Aluminiumschicht umfaßt.

7. Stabiles Hautpflegeprodukt nach einem der vorhergehenden Ansprüche, wobei die erste Zusammensetzung etwa 0,01 bis etwa 1 % eines Retinoids umfaßt, ausgewählt aus der Gruppe, bestehend aus Retinylestern, Retinol, Retinal und Gemischen hiervon.

8. Stabiles Hautpflegeprodukt nach einem der vorhergehenden Ansprüche, wobei der Retinoidverstärker Climbazol ist.

9. Verfahren zur Konditionierung der Haut, wobei das Verfahren die topische Applikation des Produktes nach einem der Ansprüche 1 bis 8 auf die Haut umfaßt.

10. Verfahren zur Simulation der Wirkung von Retinsäure auf der Haut, wobei das Verfahren die Applikation des Produktes nach einem der Ansprüche 1 bis 8 auf die Haut umfaßt.

11. Verwendung des stabilen Hautpflegeproduktes nach einem der Ansprüche 1 bis 8 bei der Herstellung eines Medikamentes zur Konditionierung der Haut.

12. Stabiles Hautpflegeprodukt nach einem der Ansprüche 1 bis 8 zur Konditionierung der Haut.

## Revendications

1. Produit de soin cutané stable comprenant :
une première composition comprenant de 0,001 % à 10 % d'un rétinoïde choisi dans le groupe constitué par des esters de rétinyle, le rétinol, le rétinal, et des mélanges de ceux-ci ;
une seconde composition comprenant de 0,0001 % à 50 % d'au moins un renforçateur de rétinoïde choisi dans le groupe constitué par le citral, le citronellol, le cocamide DEA, la damascénone, la 1,3-d,iméthyl-2-imidaaoiidànone, le géraniol, l'acide 18-bêta-glycyrrhétinique, la 8-OH-quinoléine, la N-lauroyl-sarcosine, le linalol, le linoléamide DEA, l'alpha-ionone et l'huile de lin ;
un premier compartiment pour stocker la première composition, dans lequel le premier compartiment garde la première composition hors de contact avec l'oxygène ; et
un second compartiment pour stocker la seconde composition, le premier et le seconde compartiments étant joints l'un à l'autre ; de façon à éviter la dégradation chimique de la première composition qui serait provoqué par la mise en contact avec la seconde composition.

2. Produit de soin cutané stable selon la revendication 1, dans lequel la seconde composition a au moins deux renforçateurs de rétinoide dans une quantité allant de 0,0001% à 50%.

3. Produit de soin cutané stable selon la revendication 1 ou la revendication 2, dans lequel le rétinoïde fournit un premier effet bénéfique et le renforçateur de rétinoide renforce le premier effet bénéfique.

4. Produit de soin cutané stable selon l'une quelconque des revendications 1 à 3, dans lequel le premier compartiment comprend une couche d'aluminium.

5. Produit de soin cutané stable selon l'une quelconque des revendications 1 à 4, dans lequel le second compartiment garde la seconde composition hors de contact avec l'oxygène.

6. Produit de soin cutané stable selon la revendication 5, dans lequel le second compartiment comprend une couche d'aluminium.

7. Produit de soin cutané stable selon l'une quelconque des revendications précédentes, dans lequel la première composition comprend d'environ 0,01 % à environ 1 % d'un rétinoïde choisi dans le groupe constitué par des esters de rétinyle, le rétinol, le rétinal, et des mélanges de ceux-ci.

8. Produit de soin cutané stable selon l'une quelconque des revendications précédentes, dans lequel le renforçateur de rétinoïde est le climbazole.

9. Procédé de conditionnement de la peau, le procédé comprenant l'application topique sur la peau du produit selon l'une quelconque des revendications 1 à 8.

10. Procédé pour imiter l'effet sur la peau de l'acide rétinoïque, le procédé comprenant l'application sur la peau du produit selon l'une quelconque des revendications 1 à 8.

11. Utilisation du produit de soin cutané stable selon l'une quelconque des revendications 1 à 8 dans la préparation d'un médicament pour le conditionnement de la peau.

12. Produit de soin cutané stable selon l'une quelconque des revendications 1 à 8 pour le conditionnement de la peau.
